# EUROPEAN PATENT SPECIFICATION

(11) **EP 4 370 932 B1**
(45) Date of publication and mention of the grant of the patent: **10.12.2025**
(21) Application number: 22750821.5
(22) Date of filing: 14.07.2022
(51) Int. Cl.: G01N 33/86

(54) **METHOD FOR EVALUATING RESIDUAL PLATELET THROMBOTIC POTENTIAL IN PATIENTS UNDERGOING ANTIPLATELET TREATMENT**
VERFAHREN ZUR BEURTEILUNG DES THROMBOTISCHEN POTENZIALS VON RESTTHROMBOZYTEN BEI PATIENTEN, DIE EINER THROMBOZYTENVERHÜTENDEN BEHANDLUNG UNTERZOGEN WERDEN
MÉTHODE D'ÉVALUATION DU POTENTIEL THROMBOTIQUE RÉSIDUEL DE PLAQUETTES CHEZ LES PATIENTS SOUMIS À UN TRAITEMENT ANTIPLAQUETTAIRE

(30) Priority: 15.07.2021 IT 202100018803
(43) Date of publication of application: 22.05.2024
(73) Proprietor: Centro Cardiologico Monzino S.p.A., 20121 Milano (IT); UNIVERSITA' DEGLI STUDI DI MILANO, 20122 Milano (IT)
(72) Inventor: CAMERA, Marina, 20121 MILANO (IT); BRAMBILLA, Marta, 20121 MILANO (IT); CANZANO, Paola, 20121 MILANO (IT)
(74) Representative: Bianchetti & Minoja with Trevisan & Cuonzo IPS SRL
(86) International application number: PCT/EP2022/069766
(87) International publication number: WO 2023/285607

(56) References cited:
- DATABASE EMBASE [online] 1 November 2020 (2020-11-01), BRAMBILLA M ET AL: "Inhibition of platelet-associated Tissue Factor expression by clopidogrel and aspirin", XP055905913, Database accession no. EMB-634503803
- BRAMBILLA M ET AL: "Inhibition of platelet-associated Tissue Factor expression by clopidogrel and aspirin", BLOOD TRANSFUSION 20201101 EDIZIONI SIMTI NLD, vol. 18, no. SUPPL 4, 1 November 2020 (2020-11-01), pages S460 CONF 20201105 to 20201107 Baveno - 26. Congr, ISSN: 2385-2070
- ALEIL B ET AL: "Flow cytometric analysis of intraplatelet VASP phosphorylation for the detection of clopidogrel resistance in patients with ischemic cardiovascular diseases", JOURNAL OF THROMBOSIS AND HAEMOSTASIS, BLACKWELL PUBLISHING, OXFORD, GB, vol. 3, no. 1, 1 January 2005 (2005-01-01), pages 85 - 92, XP002568520, ISSN: 1538-7933, DOI: 10.1111/J.1538-7836.2004.01063.X
- BRAMBILLA M ET AL: "ADP-induced Platelet-associated Tissue Factor Expression: Unique Involvement of the P2Y12 Receptor and Modulation by Clopidogrel Treatment", ISTH CONGRESS ABSTRACTS, 17 July 2021 (2021-07-17), pages 1 - 2, XP055902623, Retrieved from the Internet <URL:https://abstracts.isth.org/abstract/adp-induced-platelet-associated-tissue-factor-expression-unique-involvement-of-the-p2y12-receptor-and-modulation-by-clopidogrel-treatment/> [retrieved on 20220317]
- GURBEL ET AL: "Platelet Function Monitoring in Patients With Coronary Artery Disease", JOURNAL OF THE AMERICAN COLLEGE OF CARDIOLOGY, ELSEVIER, AMSTERDAM, NL, vol. 50, no. 19, 23 October 2007 (2007-10-23), pages 1822 - 1834, XP022321761, ISSN: 0735-1097, DOI: 10.1016/J.JACC.2007.07.051
- GURBEL PAUL A ET AL: "Clopidogrel Effect on Platelet Reactivity in Patients With Stent Thrombosis Results of the CREST Study", JOURNAL OF THE AMERICAN COLLEGE OF CARDIOLOGY, vol. 46, no. 10, 15 November 2005 (2005-11-15), pages 1827 - 1832, XP029656533, ISSN: 0735-1097, DOI: 10.1016/J.JACC.2005.07.056
- RENY JEAN-LUC ET AL: "Antiplatelet Drug Response Status Does Not Predict Recurrent Ischemic Events in Stable Cardiovascular Patients : Results of the Antiplatelet Drug Resistances and Ischemic Events Study", CIRCULATION, vol. 125, no. 25, 26 June 2012 (2012-06-26), US, pages 3201 - 3210, XP055901453, ISSN: 0009-7322, DOI: 10.1161/CIRCULATIONAHA.111.085464

## Description

The invention relates to a method for evaluating residual platelet thrombotic potential in patients undergoing antiplatelet treatment.

### Background to the invention

Platelets make an important contribution to haemostasis. In the event of vessel damage they adhere to the subendothelium, and rapidly activate and aggregate. Platelet recruitment to the site of the vascular lesion depends to a critical extent on the amplification systems supplied by autocrine mediators such as adenosine 5'-diphosphate (ADP) and thromboxane A2 (TXA2) (Sang Y et al., PG, Huskens D. Blood rev. 2021; 46:100733).

The contribution made by platelets to global haemostasis is not only limited to primary haemostasis. Platelets are also involved in secondary haemostasis, ie. in the coagulation process, providing (i) a procoagulant surface with exposure of phosphatidylserine (PS) due to assembly of coagulation factors, and (ii) tissue factor (TF), the key protein required to initiate coagulation (Brambilla M, et al., Thromb Haemost 2015;114:579-592). After activation by ADP, TXA2 or thrombin, the platelets expose a functionally active TF on their surface, which binds factor VIIa, thus triggering the coagulation process that gives rise to thrombin generation (Camera M. et al., Arterioscler Thromb Vasc Biol 2003;23: 1690-1696).

Increased levels of TF-positive platelets have been reported in various pathological conditions characterised by a prothrombotic phenotype, including thrombocythaemia, antiphospholipid syndrome, cancer, viral infections and cardiovascular disease (Falanga A. et al., Exp Hematol. 2007; 35:702-711; Capozzi A et al., Clin Exp Immunol. 2019; 196:59-66; Tilley RE et al., Thromb Res 2008;122:604-609; Mayne E et al., J Acquir Immune Defic Syndr.2012;59:340-346; Canzano P et al., JACC Basic Transl Sci. 2021; Brambilla M, et al., Arterioscler Thromb Vasc Biol 2008;28:947-953).

The translational implications of TF regulation by the P2Y₁₂ receptor are highly significant in cardiovascular disease, bearing in mind that treatment with P2Y₁₂ antagonists is, together with aspirin, the gold standard for secondary prevention (Benjamin EJ et al., Circulation. 2019;139: E56-E528).

TF expression in patients suffering from coronary artery disease (CAD) is inversely proportional to the degree of platelet inhibition achieved by treatment with clopidogrel, as evaluated by the VASP test. Said test, like evaluation of other platelet activation markers such as P-selectin, aGPIIbIIIa or platelet aggregation, is frequently used to evaluate residual platelet reactivity in patients undergoing treatment with clopidogrel (Gremmel T, et al., PloS one 2015;10:e0129666). Pharmacological treatment with clopidogrel is subject to a wide range of variability of individual response, and therefore requires methods for evaluating residual platelet reactivity.

Although methods for evaluating the platelet inhibition effected by P2Y₁₂ inhibitors in general and clopidogrel in particular have been developed, a significant percentage of patients, despite undergoing pharmacological treatments recognised to be effective, suffer a further thrombotic event.

Brambilla et al. (Blood Transfusion, Vol. 18 Suppl 4, 1.11.2020, page 460s) report data relating to the regulation of the expression of platelet TF by the P2Y12 receptor, the pharmacological target of clopidogrel. In support of this evidence, the data indicating that the number of TF-expressing platelets is lower in patients who respond well to clopidogrel (PRI <60%) while it is higher in patients who do not respond to the drug (PRI> 60 %).

It is also known that the P2Y₁₂ receptor regulates the exposure on the platelet surface of other proteins including GPIIbIIIa, P-selectin, CD40L (Gachet C et al, Thromb Haemost, 1997, 78 (1): 271-275 ; Hermann A et al, Platelets, 2001, 12 (2): 74-82,) contributing more generally to the regulation of platelet granule release, as well as procoagulant phospholipids such as phosphatidylserine (Leon C et al, ATVB, 23 ( 10): 1941-1947, 2003). Also in these cases, pharmacological treatment determines a reduction in the expression of these platelet activation markers and in the formation of heteroaggregates dependent on the expression of P-selectin (Storey et al, Thromb Haemost, 2002, 88 (3): 488-94 ). Finally, Van der Maijden (Thromb Haemost, 2005, 93: 1128-36) points out that in stroke patients the P2Y₁₂-mediated contribution to thrombin generation was reduced by clopidogrel treatment.

Based on the common knowledge relating to the pharmacological modulation of numerous platelet activation parameters, TF is not the only and not even the most promising candidate for the evaluation of residual thrombotic potential in patients on antiplatelet therapy. Although Gurbel et al., (J. Am. College of Cardiology, vol. 50, no. 19, 226.6.2007, 1822-1834) have hypothesized that methods of measuring coagulation and platelet interactions may be better predictors of thrombotic potential residual, other authors (Gremmel et al, Thromb Res, 2013, 132 (1): e24-30) have highlighted that "... response to antiplatelet therapy is independent of endogenous thrombin generation potential" thus providing a teaching contrary to the hypothesis formulated in 2007 by Gurbel et al. aforementioned.

### Description of the invention

It has now been discovered that evaluation of both VASP and platelet tissue factor in a blood sample from a subject treated with clopidogrel significantly improves the evaluation of residual platelet reactivity, allowing effective monitoring of the pharmacological treatment and a reliable prognosis of adverse cardiovascular events.

The experiments conducted indicate that P-selectin is an unsatisfactory marker, because it fails to distinguish between responders and non-responders on the basis of the VASP test. Although aGPIIbIIIa expression discriminates better than P-selectin, it has been found that only tissue factor (TF) evaluation detects the patients (about 10%) who, despite a good response to clopidogrel, exhibit a residual thrombotic risk, as the amount of TF associated with the platelets exceeds the median value measured in the group of poor responders. This may partly explain why the results of various clinical trials, such as the ADRIE and GRAVITAS studies, demonstrate that in patients with stable CAD, the degree of activation of GPIIbIIIa, and consequently platelet aggregation, has no significant predictive value for major adverse cardiovascular events (Reny JL, Circulation. 2012; 125:3201; Price MJ et al., Jama. 2011; 305:1097-1105; Trenk D et al., J Am Coll Cardiol 2012;59:2159-2164; Montalescot G et al., Circulation.2014;129: 2136-2143; Cayla G et al., Lancet. 2016; 388:2015-2022).

A first object of the invention is therefore a method for evaluating residual platelet thrombotic potential in a patient undergoing antiplatelet treatment with P2Y12 inhibitors selected from clopidogrel, prasugrel or ticagrelor, which comprises determination, in a sample of the patient's blood, of vasodilator-stimulated phosphoprotein phosphorylation (VASP-P) status and platelet tissue factor expression.

Simultaneous determination of VASP-P and TF on the same sample can be conducted by known methods or modifications to conventional methods within the reach of one skilled in the art. Flow cytofluorimetry, already used for VASP-P determination, is a technique that can also be conveniently used for platelet TF determination, as will be described in detail in the experimental part.

In a second aspect thereof, the invention relates to the use of a kit in the method of claims 1-3, the kit comprising a selective ligand for VASP-P, a selective ligand for platelet tissue factor, a system of detecting said ligands, a VASP phosphorylation inhibitor, diluents and fixatives.

In a preferred form thereof, the ligands are anti-TF and anti-VASP-P monoclonal mouse antibodies, and the detection system comprises fluorescent-probe-labelled anti-IgG mouse antibodies.

### DESCRIPTION OF FIGURES

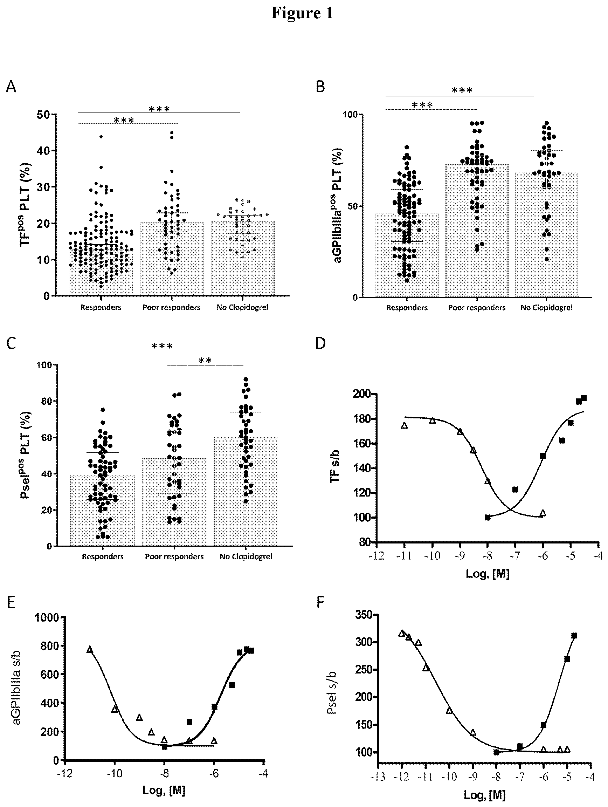

**Figure 1****: TF expression on platelets of CAD patients treated with clopidogrel.**
   The figure shows the percentage of platelets that express TF (panel A), aGPIIbIIIa (panel B) and P-selectin (panel C) following stimulation with ADP (10 µM, 15 min) in patients treated with clopidogrel (75 mg/day), divided into two groups on the basis of their platelet reactivity index (PRI) measured with the VASP test; patients with PRI<60% are classified as "responders" (n=114 patients) and those with PRI>60% as "poor responders" (n=49 patients).
   The data obtained in patients without clopidogrel (No clopido) (n=39) are shown for comparison purposes. Panels C, D and E show the ADP concentration-response curves (black squares) and AR-C69931MX antagonist inhibition curves (white triangles) relating to TF, aGPIIbIIIa and P-selectin respectively. The EC50 of the agonist was calculated with a four-parameter logistic model, while the pA2s of the antagonist were calculated as described in Materials and Methods. The results are presented as percentage expression of the protein compared with baseline (s/b). The experiments were conducted at least three times. ** p<0.01, *** p<0.001.

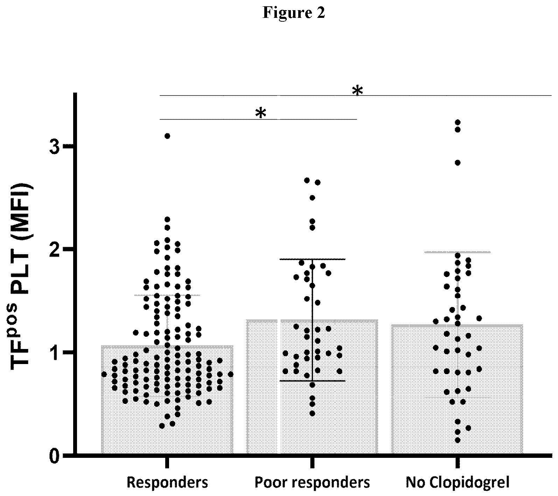

**Figure 2****: TF expression on platelets of CAD patients treated with clopidogrel.**
   The figure shows the amount of TF expressed by the platelets following stimulation with ADP (10 µM, 15 min) in patients treated with clopidogrel (75 mg/day), divided into two groups on the basis of their platelet reactivity index (PRI) measured with the VASP test: patients with PRI<60% are classified as "responders" (n=144 patients) and those with PRI>60% as "poor responders" (n=49 patients).
   The data obtained in patients without clopidogrel (No clopido) (n=39) are shown for comparison purposes. The results are reported as mean fluorescence intensity (MFI). * p<0.05.

The invention will now be described in detail in the following experimental part, provided by way of example.

### MATERIALS AND METHODS

### Study population

The *in vitro* experiments were conducted on 10 healthy volunteers (n=5 males and n=5 females; average age 39 ± 6) who had not taken antiplatelet medicaments in the 10 days before the blood sample was taken. The effect of clopidogrel on TF expression was evaluated on 193 patients suffering from coronary artery disease (CAD) with myocardial infarction with non-ST elevation. 39 patients not subjected to clopidogrel treatment have also been recruited as a control group. The clinical characteristics of the patients enrolled are set out in Table 1.

**Table 1: Clinical characteristics of patients included in the study**

| | **Clopidogrel-treated CAD Patients (n=193)** | **No Clopidogrel-treated CAD Patients (n=39)** | **P value** |
|---|---|---|---|
| **Male** | **151 (78)** | **35** (89) | 0.100 |
| **Age, years** | **73 ± 11** | **72 ±** 4.8 | 0.766 |

| **Major Cardiovascular risk factors** | | | |
|---|---|---|---|
| Hypertension | 112 (58) | 27 (69) | 0.262 |
| Diabetes mellitus | 45 (23) | 5 (14) | 0.212 |
| Hyperlipidemia | 44 (23) | 30 (78) | <0.0001 |
| Current smoker | 22 (11) | 10 (25) | 0.943 |

| **Medications** | | | |
|---|---|---|---|
| Clopidogrel | 193 (100) | 0 (0) | <0.0001 |
| Aspirin | 184 (95) | 39 (100) | 0.853 |
| RAAS inhibitors | 106 (55) | 10 (47) | 0.409 |
| Calcium-channel blockers | 47 (24) | 11 (28) | 0.761 |
| Beta-blockers | 109 (56) | 29 (75) | 0.058 |
| Nitrates | 27 (14) | 5 (14) | 0.951 |
| Hypolipidemics | 118 (61) | 36 (92) | 0.0003 |
| Proton-pump inhibitors | 110 (57) | 17 (44) | 0.174 |

| | | | |
|---|---|---|---|
| Data expressed as mean±sd or n (%). | | | |

All patients had been treated with clopidogrel (75 mg/day) for at least 7 days. The exclusion criteria were: age > 80 years, valvular heart disease, atrial fibrillation, thyrotoxicosis, history of haemorrhagic diathesis, platelet disorder or thrombocytopenia, anticoagulant or thrombolytic treatment. A subject with severe congenital P2Y₁₂ deficiency (male, age 75 years) was also studied.

### Blood drawing and platelet isolation

Whole blood (WB) samples were taken with a 19 gauge needle without venous stasis in tubes containing sodium citrate (0.129 M, 1/10 volume/volume) (Vacutainer, Becton Dickinson), discarding the first 4 ml. The blood samples were then processed within 15 minutes of sample-drawing. The WB was centrifuged (room temperature, without centrifuge brake) at 100 g for 10 minutes to isolate the platelets. The platelet-rich plasma (PRP) was transferred to a new test tube and analysed with the Sysmex XE-2100 Automated Hematology Analyzer to determine the platelet count, mean platelet volume (MPV), platelet distribution dispersion (PDW), platelet recovery (defined as the percentage of the platelet count in the PRP to 100% of the number of platelets in the WB), and leukocyte contamination. Only the platelet preparations free of leukocyte contamination and with platelet recovery ≥70% were used for the subsequent thrombin generation analysis.

### Flow cytometry analysis

Surface expression of platelet activation markers was analysed by flow cytometry as previously described (Brambilla M, et al., Arterioscler Thromb Vasc Biol. 2008;28(5):947-953). Briefly, the samples were incubated/labelled with saturating concentrations of mouse monoclonal antibodies recognizing human tissue factor (HTF1; Thermo Fisher), P-selectin (APC; Becton Dickinson), PAC1 (FITC; Becton Dickinson), CD36 (PE; Becton Dickinson) and CD154 (FITC; Becton Dickinson). For the analysis of intracellular expression of TF, the cells were fixed for 2 hours with 1% paraformaldehyde, permeabilised for 10 min with an 0.1% solution of Triton X-100 PBS, and labelled for 15 min at room temperature in the dark with saturating concentrations of anti-TF and CD61 antibodies (PerCP; Becton Dickinson). An aspecific antibody (Becton Dickinson) and the labelling only with the secondary antibody AlexaFluor^{®} 633 labelled IgG ( Thermo Fisher) were used as controls in all the experiments to quantify the aspecific labelling signal. Before use, all antibodies were centrifuged at 17,000 rpm for 5 min at 4°C to remove any aggregate. A total of 10,000 platelets per sample were acquired with a Gallios flow cytofluorimeter (Beckman Coulter), equipped with four solid-state lasers at 488 nm, 638 nm, 405 nm and 561 nm. Flow-check Pro Fluorospheres (Beckman Coulter) were used daily, according to the manufacturer's instructions, to monitor the performance of the cytofluorimeter. All the data were analysed with Kaluza analysis software v1.5 (Beckman Coulter) and reported as percentage of positive cells or MFI (mean fluorescence intensity) of platelets positive to the activation marker. P2Y₁₂-dependent phosphorylation of the VASP protein was analysed with a commercial kit according to the manufacturer's instructions (PLT VASP/P2Y₁₂ test kit; Stago), and it has been expressed as percentage platelet reactivity index (PRI).

### Statistical analysis

The results are expressed as mean ± standard deviation (SD), and analysed with Student's t-test or the Mann Whitney U-test. The continuous variables were compared between groups by one-way repeated analysis of variance (ANOVA) followed by Dunnett's post hoc analysis. A p-value of 0.05 was considered statistically significant. The analyses were conducted with the SPSS statistical package (v9.4). The concentration-response curves were evaluated with GraphPad Prism 8. The pA2s values were calculated according to the equation written as described in Prism 8. The standar errors of the parametersare expressed as percentage coefficient of variation (%CV), and calculated by means of analysis of at least three different, independent experiments. All curves shown are generated by informatics methods.

**The exposure of TF associated with platelets in CAD patients treated with clopidogrel is negatively associated with P2Y₁₂ inhibition.**

The association between platelet TF expression and degree of P2Y₁₂ inhibition, measured by the VASP phosphorylation assay in CAD patients treated with clopidogrel, was analysed. The degree of VASP phosphorylation is associated with the levels of the active metabolite of clopidogrel (Liang Y et al., J Thromb Thrombolysis 2012;34:429-436), allowing patients to be classified as good responders when the platelet reactivity index (PRI) is <60% (Aleil B et al., J Thromb Haemost. 2005;3(1):85-92). The results demonstrate that platelet exposure of TF induced by ADP in CAD patients treated with clopidogrel is negatively associated with P2Y₁₂ inhibition. In patients with a suboptimal response to pharmacological treatment, ie. with a PRI value >60%, inhibition of TF exposed to stimulation with 10 µM ADP was significantly less than that measured in the good responders (PRI value <60%), and not statistically different from that measured in the subjects not treated with clopidogrel (Fig. 1A). As expected, expression of activated glycoprotein IIbIIIa (aGPIIbIIIa), measured by the PAC1 bond, was highest in the poor responders (Fig. 1B). Conversely, clopidogrel significantly inhibited P-selectin expression to the same extent in all the CAD patients, regardless of the degree of VASP phosphorylation (Fig. 1C). These results are consistent with the finding that the power of AR-C69931MX (pA2) to inhibit P-selectin expression, estimated *in vitro,* is 150 and 6 times greater than that required to inhibit TF and aGPIIbIIIa (9.33±2.1% CV, 10.7±0.29% CV and 11.5±3.1% CV for TF, aGPIIbIIIa and P-selectin respectively; Fig. 1D-F).

23 of the 144 good responders, namely 15% of those patients, had a percentage of TF-positive platelets exceeding the average number measured in the group of poor responders. Said percentage actually doubles (to 38%) if the average amount of protein expressed on the surface of the platelets is taken into account (Fig. 2). This result demonstrates the presence of a sub-group of patients with increased prothrombotic potential despite an apparently good pharmacological response to clopidogrel as measured by the VASP test.

Taken as a whole, these results demonstrate that in patients treated with clopidogrel, inhibition of TF and aGPIIbIIIa expression following stimulation with ADP, but not P-selectin inhibition, is directly associated with the amount of platelet inhibition measured by the VASP assay. This suggests that a concentration of medicament sufficient to inhibit P-selectin expression can only partly reduce exposure of TF and aGPIIbIIIa, and therefore the platelet prothrombotic potential.

Only P2Y₁₂ antagonism completely prevented TF exposure on the cell surface, thus influencing the kinetics of thrombin generation. Said effect was observed in both *in vitro* and *ex vivo* experiments on platelets of patients treated with clopidogrel. Interestingly, the degree of P2Y₁₂ inhibition required to prevent the expression of TF associated with platelets was much higher than that required to prevent P-selectin expression.

TF expression in CAD patients is inversely proportional to the degree of platelet inhibition by treatment with clopidogrel, as evaluated by the VASP assay.

The data also demonstrate that translocation of TF on the platelet membrane following stimulation with ADP is also a P2Y₁₂-dependent mechanism, because only the specific antagonist AR-C69931MX, not the specific antagonist of the P2Y1 receptor, was able to inhibit it.

The therapeutic benefits of antiplatelet treatment with clopidogrel are therefore presumably partly due to inhibition of platelet TF expression. In this context, concomitant evaluation of VASP and TF in patients treated with P2Y₁₂ inhibitors improves the evaluation of residual platelet reactivity.

## Claims

1. A method for assessment of residual platelet thrombotic potential in a patient undergoing antiplatelet treatment with a P2Y₁₂ receptor inhibitor selected from clopidogrel, prasugrel or ticagrelor, comprising determination in a patient's blood sample of vasodilator-stimulated phosphoprotein phosphorylation (VASP-P) status and platelet tissue factor (TF) expression.

2. A method according to claim 1 wherein the P2Y₁₂ receptor inhibitor is clopidogrel.

3. A method according to any one of claims 1 to 2 wherein VASP-P and TF are determined by flow cytometry.

4. Use of a kit in the method of claims 1-3, the kit comprising a selective ligand for VASP-P, a selective ligand for platelet tissue factor, a detection system for said ligands, a VASP phosphorylation inhibitor, diluents and fixatives.

5. Use of a kit according to claim 4, wherein said ligands are anti-TF and anti-VASP-P monoclonal mouse antibodies.

6. Use of a kit according to claim 5, wherein the detection system comprises fluorescent-probe-labelled anti-IgG mouse antibodies.

7. Use of a kit according to claim 4 or 5, wherein the VASP phosphorylation inhibitors comprise ADP and PGE1.

## Patentansprüche

1. Verfahren zur Beurteilung des verbleibenden thrombotischen Potenzials der Thrombozyten bei einem Patienten, der eine Thrombozytenaggregationshemmer-Behandlung mit einem P2Y₁₂-Rezeptorhemmer erhält, der aus Clopidogrel, Prasugrel oder Ticagrelor ausgewählt ist, umfassend die Bestimmung des Status der vasodilatatorisch stimulierten Phosphoproteinphosphorylierung (VASP-P) und der Expression des Thrombozyten-Gewebefaktors (TF) in einer Blutprobe des Patienten.

2. Verfahren nach Anspruch 1, wobei der P2Y₁₂-Rezeptorhemmer Clopidogrel ist.

3. Verfahren nach einem der Ansprüche 1 bis 2, wobei VASP-P und TF durch Durchflusszytometrie bestimmt werden.

4. Verwendung eines Kits in dem Verfahren nach den Ansprüchen 1 bis 3, wobei das Kit einen selektiven Liganden für VASP-P, einen selektiven Liganden für den Gewebefaktor von Thrombozyten, ein Nachweissystem für die genannten Liganden, einen VASP-Phosphorylierungshemmer, Verdünnungsmittel und Fixiermittel umfasst.

5. Verwendung eines Kits nach Anspruch 4, wobei die Liganden monoklonale Maus-Antikörper gegen TF und VASP-P sind.

6. Verwendung eines Kits nach Anspruch 5, wobei das Nachweissystem fluoreszenzmarkierte Anti-IgG-Maus-Antikörper umfasst.

7. Verwendung eines Kits nach Anspruch 4 oder 5, wobei die VASP-Phosphorylierungshemmer ADP und PGE1 umfassen.

## Revendications

1. Procédé d'évaluation du potentiel thrombotique plaquettaire résiduel chez un patient sous traitement antiplaquettaire par un inhibiteur du récepteur P2Y₁₂ choisi parmi le clopidogrel, le prasugrel ou le ticagrélor, comprenant la détermination, dans un échantillon de sang du patient, du statut de phosphorylation des phosphoprotéines stimulées par les vasodilatateurs (VASP-P) et de l'expression du facteur tissulaire (TF) plaquettaire.

2. Procédé selon la revendication 1, dans lequel l'inhibiteur du récepteur P2Y₁₂ est le clopidogrel.

3. Procédé selon l'une quelconque des revendications 1 à 2, dans lequel VASP-P et TF sont déterminés par cytométrie en flux.

4. Utilisation d'un kit dans le procédé des revendications 1 à 3, le kit comprenant un ligand sélectif de VASP-P, un ligand sélectif du facteur tissulaire plaquettaire, un système de détection desdits ligands, un inhibiteur de la phosphorylation de VASP, des diluants et des fixateurs.

5. Utilisation d'un kit selon la revendication 4, dans laquelle lesdits ligands sont des anticorps monoclonaux de souris anti-TF et anti-VASP-P.

6. Utilisation d'un kit selon la revendication 5, dans laquelle le système de détection comprend des anticorps de souris anti-IgG marqués par une sonde fluorescente.

7. Utilisation d'un kit selon la revendication 4 ou 5, dans laquelle les inhibiteurs de la phosphorylation de VASP comprennent l'ADP et la PGE1.
